(19) Europäisches Patentamt
European Patent Office
Office européen des brevets

(11) Veröffentlichungsnummer : **0 309 912 B1**

# (12) EUROPÄISCHE PATENTSCHRIFT

(45) Veröffentlichungstag der Patentschrift : **15.05.91 Patentblatt 91/20**

(51) Int. Cl.[5] : **C07D 307/92**, // C11B9/00

(21) Anmeldenummer : **88115568.3**

(22) Anmeldetag : **22.09.88**

## (54) Verfahren zur Herstellung von alkylierten Dodecahydro-naphtho[2,1-b]-furanen.

Die Akte enthält technische Angaben, die nach dem Eingang der Anmeldung eingereicht wurden und die nicht in dieser Patentschrift enthalten sind.

(30) Priorität : **30.09.87 DE 3732954**

(43) Veröffentlichungstag der Anmeldung : **05.04.89 Patentblatt 89/14**

(45) Bekanntmachung des Hinweises auf die Patenterteilung : **15.05.91 Patentblatt 91/20**

(84) Benannte Vertragsstaaten : **CH DE FR GB LI NL**

(56) Entgegenhaltungen :
**EP-A- 0 165 458**
**EP-A- 0 204 009**
**Synthesis 983-985 (1985)**

(73) Patentinhaber : **BASF Aktiengesellschaft**
**Carl-Bosch-Strasse 38**
**W-6700 Ludwigshafen (DE)**

(72) Erfinder : **Gramlich, Walter, Dr.**
**Auf der Hoehe 11**
**W-6803 Edingen-Neckarhausen (DE)**
Erfinder : **Hickmann, Eckhard, Dr.**
**Kantstrasse 23**
**W-6701 Dannstadt-Schauernheim (DE)**
Erfinder : **Becker, Rainer, Dr.**
**Im Haseneck 22**
**W-6702 Bad Duerkheim (DE)**
Erfinder : **Lauterbach, Gerald, Dr.**
**Breite Strasse 63**
**W-6700 Ludwigshafen (DE)**

**EP 0 309 912 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Eine Verbindung mit dem Grundgerüst des Dodecahydro-naphtho[2,1-b]furans der Formel I

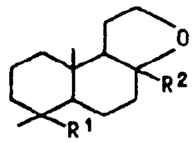

(I)

in der $R^1$ und $R^2$ gleiche Bedeutung besitzen und für eine Methyl-Gruppe stehen, stellt in Form eines Enantiomeren ([3aR-(3aα,5aβ,9aα,9bβ)]) einen seit Jahrzehnten in der Parfümerie verwendeten Ambra-Riechstoff (Ambrox® der Fa. Firmenich ; Ambroxan® der Fa. Henkel) dar.

Dieser Ambra-Riechstoff wurde inzwischen neben seinem Vorkommen in der Ambra-Tinktur des Pottwales ebenfalls in Muskateller-Salbei-Öl (Salvia Sclarea L.), im Labdanum-Öl (Cistus labdaniferus L.) sowie in Cypressen-Öl (Cupressus semperoirens L.) nachgewiesen (vgl. G. Ohloff in Fragrance Chemistry, Academic Press, 1982, S. 543). Er ruft den in Parfüm-Kompositionen begehrten "Ambra-Effekt" hervor, der auch in großer Verdünnung wirksam ist und sorgt darüber hinaus für eine ausgezeichnete Fixierung auch zarter, blumiger Düfte. Er wird hauptsächlich in teuren Parfümölen eingesetzt.

Die vier Diastereomeren dieses Ambra-Riechstoffs (Formel I, $R^1$, $R^2 = CH_3$)

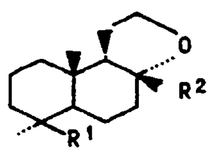

(Ia)

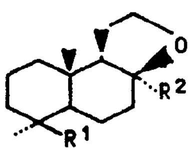

(Ib)

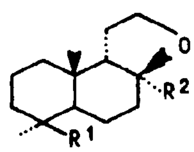

(Ic)

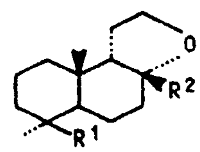

(Id)

Ia-Id unterscheiden sich deutlich voneinander. So ist z.B. der Geruch des sogenannten "Isoambrox" (Ib) über hundertmal schwächer als der der Verbindung der Formel Ia (vgl. G. Ohloff et al. i. Helv. Chimica Acta, Vol. 68 (1985), S. 2022 bis 2029) und der Geruch des sogenannten "Norisoambrox" (Formel Ib mit $R^1 = H$ und $R^2 = CH_3$) über 500 ml schwächer als der der Verbindung der Formel Ia.

Der Ambra-Riechstoff der Formel Ia ($R^1$, $R^2 = CH_3$) bzw. die Diastereomeren-Gemische der Formeln Ia bis Id lassen sich sowohl partialsynthetisch aus Ambrein oder Diterpenen vom Labdan-Typ (z.B. Sclareol, Manool) als auch durch Totalsynthese herstellen (vgl. Übersicht von E.-J. Brunke, Dragoco-Report, 11/12-1979, S. 276 ff).

Im technischen Maßstab wird der Ambra-Riechstoff hauptsächlich durch oxidativen Abbau (Chromsäure, Permanganat, Ozon) von Sclareol, einem Inhaltsstoff des Muskateller Salbei Concretes, hergestellt. Das als Abbau-Produkt erhaltene Sclareolid wird nach einer Lithiumalanat- oder Natriumboranat-Reduktion in ein 1,4-Diol, ("Ambroxdiol") übergeführt, das anschließend cyclisiert wird (vgl. G. Ohloff, Fortschr. Chem. Forsch. 12/2, 185 ff (1969).

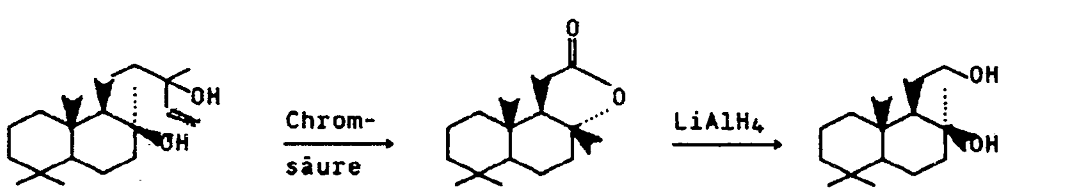


(-)Sclareol

(+)Sclareolid

"Ambroxdiol"

Ia - Id

Aufgrund der Tatsache, daß unter den vier Diastereomeren die Verbindung der Formel Ia die größte olfaktorische Bedeutung besitzt, wurden in der Vergangenheit zahlreiche Verfahren entwickelt, die versuchten den

letzten Schritt, die Cyclisierung, hochselektiv durchzuführen.

So beschrieben V.E. Sibiertseva et al. in Maslo-Zhir, Prom.-st., 1979 (12), S. 25 bis 26, die Cyclisierung des "Ambroxdiols" in Gegenwart von p-Toluolsulfonsäure. Nachteilig an dem Verfahren ist die im sauren Medium nicht zu vermeidende Dehydratisierung der tertiären Hydroxygruppe, die zu Selektivitätsverlusten führt. Außerdem sind die angegebenen Ausbeuten von 55 bis 60 % bei einem technischen Verfahren unbefriedigend, insbesondere unter Berücksichtigung des teuren Edukts.

Die Verwendung von p-Toluolsulfonsäure wird auch in den Verfahren der russischen Patente SU 345-153 (von 1968), SU 910-561 (von 1980) sowie SU 529-166 (von 1975) beansprucht.

In dem Verfahren gemäß einem älteren Patent der Fa. Firmenich (DP 860 214 von 1949) wurden als Katalysatoren Naphthalinsulfonsäure (78% Ausbeute) sowie Aluminiumoxid (50% Ausbeute) beschrieben. Auch hierbei wurden die oben aufgeführten Nachteile, wie schlechte Selektivität, sowie unbefriedigende Ausbeuten beobachtet.

Gemäß dem Verfahren des spanischen Patents 432 813 (von 1976) wurde die Cyclisierung des "Ambroxdiols" mit Schwefelsäure durchgeführt ; die hierbei erzielten Ausbeuten lagen beinur 29%.

R.C. Cambie et al beschrieben in Aust. J. Chem. 24 (1971), S. 583 bis 591 sowie S. 2365 bis 2377, die Cyclisierung des "Ambroxdiols" mittels p-Toluolsulfonylchlorid in Pyridin mit 80% Ausbeute. Hauptnachteile sind in diesem Falle der Einsatz des unangenehm riechenden Pyridins im Hinblick auf die Qualitätseinstellung des erhaltenen Riechstoffes sowie die relativ langen Reaktionszeiten der Umsetzung. Darüber hinaus ist bei der wäßrigen Aufarbeitung eine Pyridin-Rückführung durch dessen Wasserlöslichkeit unumgänglich, was zu einer Kostensteigerung führt.

Cambie verwendete bei einer zweiten Cyclisierungsmethode Schwefelsäure als Katalysator. Nachteilig sind hierbei die lange Reaktionszeit (>3 Tage) sowie die schlechten Ausbeuten (43% der Theorie).

Auch bei dem vom Consertium Elektrochemie in der DOS 3 240 054 (von 1982) beschriebenen Cyclisierungsverfahren mit $POCl_3$ arbeitete man in wasserfreiem Pyridin und man mußte daher die oben beschriebenen Nachteile in Kauf nehmen. Die Ausbeuten betrugen nur 65% d. Theorie.

P.F. Vead and N.D. Unger beschrieben in "Synthesis", 1983, S. 216 bis 218 eine Cyclisierungsmethode, bei der als Reagens Trimethylchlorsilan in Dimethylsulfoxid (DMSO) eingesetzt wurde ; die hierbei angegebenen Ausbeuten betragen 85%. Wie aus dem hierin aufgeführten experimentellen Beispiel hervorgeht, stellt diese Methode nur ein Laborverfahren dar (mg-Ansätze). Für die Technik von Nachteil ist der Einsatz des DMSO, das nach der wäßrigen Aufarbeitung aufgrund seiner Wasserlöslichkeit (Abwasserprobleme) aufwendig recyclisiert werden muß. Ein entscheidender Nachteil ist darüber hinaus die Notwendigkeit einer Reinigung des nach der Reaktion erhaltenen rohen Ambra-Riechstoffs auf eine sowohl chemisch als auch olfaktorisch akzeptierbare Qualität. Die chemische Reinigung erfolgte durch kostspielige Säulenchromatographie, wobei eine vollständige DMSO-Entfernung meist schwierig ist. Ein weiteres Problem ist das technisch häufig schwierige Handling von Trimethylchlorsilan, welches sehr korrusiv wirkt und außerdem toxikologisch bedenklich ist.

Die gleiche Cyclisierungsmethode wird auch im russischen Patent SU 988 817 (von 1980) beschrieben.

Weiterhin ist aus EP-A-204 009 ein Verfahren zur Herstellung von Ambrox® bekannt, bei dem Sclareol auf biotechnischem Wege in das sogenannte "Ambroxdiol" überführt und dieses zum Dodecahydro-3a,6,6,9a-tetramethylnaphtho[2,1-b]furan der Formel I cyclisiert wird. Die Cyclisierung wird mit einem Arylsulfonylchlorid in Gegenwart von sauren Verbindungen, wie HCl und sauren Ionenaustauschern oder in Gegenwart von Basen, wie Pyridin und NaOH vorgenommen. Bei der hier beschriebenen Cyclisierung in saurem Milieu werden schlechte Ausbeuten erzielt. Die Nachteile einer Cyclisierung in Gegenwart von organischen Basen, wie Pyridin im technischen Maßstab, wurden oben ausführlich erläutert.

Relativ gut gelingt die Cyclisierung mit Sulfonylchloriden in organischen Lösungsmitteln und in Gegenwart von Alkalihydroxiden. Nachteilig hierbei sind jedoch die relativ langen Reaktionszeiten ; die noch ungenügenden Reinheiten der erhaltenen Verbindungen der Formel I sowie die teilweise hohe Reaktionstemperatur, die zu Selektivitätsverlusten führen kann (vgl. Beispiel 9).

Es bestand somit die Aufgabe, ein hochselektives Cyclisierungsverfahren aufzufinden, das technisch problemlos durchführbar ist und ohne die beschriebenen Nachteile zu einem olfaktorisch geeigneten Produkt führt.

Es wurde nun überraschenderweise gefunden, daß sich die Cyclisierung des "Ambroxdiols" ohne die aufgeführten Nachteile der bisherigen Verfahren dadurch erzielen läßt, daß man das Diol in einem organischen Lösungsmittel mit einem Sulfonsäurechlorid und wäßriger Alkalihydroxide in Gegenwart eines Phasentransfer-Katalysators (PTK) cyclisiert.

Unseres Wissens ist die Cyclisierung eines 1,4-Diols mit einem Sulfonsäurechlorid das erste Beispiel einer Tetrahydrofuran-Synthese in Gegenwart eines Phasentransfer-Katalysators. Übersichten über Reaktionen in Gegenwart von Phasentransfer-Katalysatoren : E.V. Dehmlow und S.S. Dehmlow : Phase Transfer Catalysis, Verlag Chemie, 1980 ; insbesondere E.V. Dehmlow in Angew. Chemie 89 (1977), Seiten 521f und Angew. Chemie 86 (1974), Seiten 187f).

Gegenstand der Erfindung ist dem entsprechend ein Verfahren zur Herstellung von alkylierten Dodecahydro-naphtho[2,1-b]furanen der allgemeinen Formel I

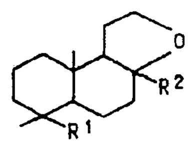

I

in der $R^1$ und $R^2$ die gleiche Bedeutung haben und für Wasserstoff oder eine Methylgruppe stehen, durch Umsetzung eines alkylierten 2-Hydroxy-decahydro-napthalin-1-ethanols der Formel II

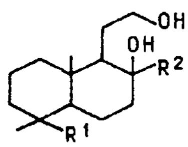

II

in der $R^1$ und $R^2$ die obengenannte Bedeutung besitzen, mit einem Sulfochlorid in einem organischen Lösungsmittel und in Gegenwart basischer Katalysatoren, dadurch gekennzeichnet, daß man die Cyclisierung in Gegenwart von konzentrierten wäßrigen Alkalihydroxiden und eines Phasentransferkatalysator durchführt.

Die bei dem erfindungsgemäßen Verfahren überraschenderweise erzielte Selektivität liegt bei über 95% bei gleichzeitig vollständigem Umsatz ; diese gegenüber dem besten Stand der Technik über 10%ige Ausbeutesteigerung stellt bei den hohen Preisen für die Ausgangsverbindung eine bedeutende Kostenreduzierung dar.

Darüber hinaus ist das in der Technik als Ausgangsverbindung zumeist eingesetzte Sclareol nur in beschränktem Maße verfügbar und unterliegt als Naturprodukt häufig großen Mengenschwankungen bei der Ernte, die durch das Klima verursacht sind. Durch die über 10%ige Ausbeutesteigerung wird daher weniger des teuren Edukts Sclareol benötigt.

Ein weiterer, ganz wesentlicher Fortschritt gegenüber den bisherigen, in Gegenwart von Pyridin durchgeführten Ringschluß-Reaktionen ist die wesentlich einfachere Aufarbeitung und Reinigung des Produktes zu einem olfaktorisch akzeptierbaren Ambra-Riechstoff. Da die Ambra-Note durch einen warmen, zarten, balsamischen und erotisierenden Duft geprägt ist, treten nämlich auch kleine Verunreinigungen im Riechstoff stark hervor.

Die bei dem erfindungsgemäßen Verfahren erzielten Selektivitäten sind sehr gut.

Daher bereitet auch die Reinigung des nach dem neuen erfindungsgemäßen Verfahren hergestellten Produktes keinerlei Probleme.

Für das erfindungsgemäße Verfahren lassen sich zahlreiche Phasentransfer-Katalysatoren (allg. Übersicht vgl. V. Dehmlow, Angew. Chemie, 89 (1977), Seite 521 bis 533) verwenden. Besonders bevorzugte Phasentransferkatalysatoren (PTK) sind :

1. Tetraalkylammoniumsalze der allgemeinen Formel IV

$$R^4-\overset{\displaystyle R^5}{\underset{\displaystyle R^7}{\overset{|}{\underset{|}{N^{\oplus}}}}}-R^6 \qquad X^{\ominus}\ , \qquad \text{(IV), in der}$$

$R^4$, $R^5$, $R^6$ und $R^7$ untereinander gleich oder verschieden sein können und für Alkylreste mit 1 bis 22 Kohlenstoffatomen oder für funktionelle Gruppen, wie Hydroxyl–, Carbonamid– oder Ethergruppen enthaltende Alkylreste mit bis zu 25 Kohlenstoffatomen wie Methyl-, Ethyl–, (Iso)propyl–, Butyl–, Octyl–, Dodecyl–, $C_{16}H_{33}$–, Hydroxy(iso)-propyl- oder

$$C_{17}H_{33}\underset{\underset{\displaystyle O}{\|}}{C}-NH-CH_2-CH_2-CH_2- \ ,$$

sowie für Phenylreste oder phenylsubstituierte Alkylreste (wie z.B. Benzyl-) mit bis zu 20 Kohlenstoffato-

men stehen.

und $X^{\ominus}$ für ein Anion einer Säure wie $J^-$, $Cl^-$, $Br^-$, $(HSO_4)^-$, $(CN)^-$, $(BF_4)^-$ steht ; insbesondere das sehr preiswerte Trimethylbenzyl-ammoniumchlorid, das in Form seiner 50%igen wäßrigen Lösung eingesetzt werden kann, sowie Tricaprylmethylammoniumchlorid ; und

2. Tetraalkylphosphoniumsalze der allgemeinen Formel V

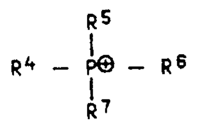 $X^{\ominus}$ , (V)

worin $R^4$, $R^5$, $R^6$, $R^7$ sowie $X^{\ominus}$ die für die Formel (IV) angegebene Bedeutung haben, insbesondere das Tri-n-octyl-methylphosphoniumiodid. Weiterhin geeignet sind auch Gemische der oben genannten PTK sowie Träger-fixierte PTK.

Die PTK werden für das erfindungsgemäße Verfahren in Mengen von 0,1 bis 1, vorzugsweise 0,3 bis 0,5 Mol pro Mol Diol eingesetzt.

Die für die PTK-Reaktion geeigneten Lösungsmittel hängen von der Löslichkeit des einzusetzenden Diols ab ; geeignet sind u.a. Dichlormethan, Tetrahydrofuran, Dioxan, Benzol, Toluol sowie Xylol.

Als basische Katalysatoren haben sich konzentrierte wäßrige Alkalihydroxide, insbesondere wäßrige Natron- und Kalilauge als geeignet erwiesen. Die Menge der eingesetzten Lauge ist wenig kritisch. Sie kann zwischen 2 und 8 Mol pro Mol des eingesetzten Diols schwanken. Für eine besonders schnelle Cyclisierung hat sich ein etwa molarer Überschuß der Lauge, bezogen auf das Diol, als optimal erwiesen (Gesamtreaktionszeit ~1 h). Verringert man die eingesetzte Menge Lauge, so wird die Reaktionsgeschwindigkeit verlangsamt. Eine Erhöhung der Laugenmenge ist unwirtschaftlich da die schon kurze Reaktionszeit nicht mehr beschleunigt werden kann.

Als Sulfochloride haben sich die für Substitutionsreaktionen bekannten Reagenzien wie Methylsulfochlorid, Ethylsulfochlorid, Benzolsulfochlorid, p-Tolylsulfochlorid, p-Chlor- bzw. p-Brom-phenylsulfochlorid und p-Nitro-phenylsulfochlorid bestens bewährt, d.h. Sulfochloride der allgemeinen Formel III

$$R^3\text{-}SO_2Cl \quad \text{(III)}$$

in der $R^3$ für einen $C_1$-$C_3$-Alkylrest oder einen Phenylrest steht, der in para-Stellung mit einer Methylgruppe, einem Chlor- oder Bromatom oder einer Nitrogruppe substituiert sein kann.

Die als Ausgangsstoffe eingesetzten Diole der allgemeinen Formel II lassen sich nach bekannten Literaturmethoden erhalten (vgl. G. Ohloff et al., Helv. Chim. Acta, 68 (1985), S. 2022 bis 2029).

Mit Hilfe des erfindungsgemäßen Verfahrens lassen sich die als Ambra-Riechstoffe begehrten Dodecahydronaphtho[2,1-b]furane der allgemeinen Formel I auf einfache Weise in relativ kurzen Reaktionszeiten mit Selektivitäten von bis zu 95%, bei vollständigem Umsatz, herstellen.

Die folgenden Beispiele sollen das erfindungsgemäße Verfahren näher beschreiben.

Beispiel 1

Herstellung von [3aR-(3aα,5aβ,9aα,9bβ)]-dodecahydro-3a,6,6,9a-tetramethylnaphtho[2,1-b]furan (Formel Ia ($R^1$, $R^2$ = Methyl).

142 g (0,56 mol) Decahydro-2-hydroxy-2,5,5,8a-tetramethyl-1-naphthalinethanol, hergestellt aus Sclareolid (Dodecahydro-3a,6,6,9a-tetramethylnaphtho[2,1-b]furan-2-on, Fp. 124°C, $[\alpha]_D^{20}$ = + 48°C, Fa. Reynolds/USA) nach M. Hinder u. M. Stoll in Helv. Chimica Acta 36 (1953), S. 1995 bis 2008 (mit Fp. 132°C) wurde in 2110 g (2434 ml) Toluol gelöst. Anschließend wurden bei 25°C zunächst 200 ml (304 g) einer 50%igen Natronlauge (3,8 mol), danach 86 ml einer 50%igen wäßrigen Lösung von Benzyltrimethylammoniumchlorid (0,2 mol) zugetropft. Unter leichter Kühlung wurde innerhalb von einer Stunde (1 h) 105 g (0,6 mol) Benzolsulfonsäurechlorid zugetropft, das Reaktionsgemisch 2 h nachgerührt (nach DC-Analytik trat ein vollständiger Umsatz ein).

Zur Aufarbeitung wurde 500 ml Wasser zugetropft, 15 Min nachgerührt, 1 h absitzen gelassen und danach die Phasentrennung vorgenommen.

Die untere Phase wurde abgetrennt, die obere toluolhaltige Phase wurde noch einmal mit 250 ml Wasser gewaschen und danach das Toluol bei 400 mbar abdestilliert. Der verbleibende Rückstand wurde unter stark

vermindertem Druck destilliert (Kp. 100°C/0,006 mbar).

Man erhielt 126,2 g [3aR-(3aα,5aβ,9aα,9bβ]-dodecahydro-3a,6,6,9a-tetramethylnaphtho[2,1-b]-furan, was einer Ausbeute von 95,5% entspricht (Reinheit laut GC : 99,9% ; Fp. 76 bis 77°C,$[\alpha]_D^{25}$ = 26,3°).

Beispiel 2

Herstellung von Isoambrox (Formel Ib mit $R^1$, $R^2 = CH_3$)

Durch Isomerisierung und anschließende Lithiumalanat-Reduktion von Sclareolid wurde nach G. Ohloff in Helv. Chim. Acta, 68 (1985), S. 2022 bis 2029, ein diastereomeres Decahydro-2-hydroxy-2,5,5,8a-tetramethyl-1-naphthalinethanol (Fp. 191°C) erhalten. 142 g dieses Diols wurden in 2110 g Toluol gelöst, die Lösung mit 120 g (3 mol) Natriumhydroxid in 120 ml Wasser und danach mit 48 g (0,15 mol) Tetrabutylammoniumbromid versetzt. Innerhalb von 1 h wurden dann 101 g (0,5 mol) p-Toluolsulfochlorid zugetropft, das Reaktionsgemisch 2 h nachgerührt, dann mit 500 ml Wasser versetzt und anschließend 10 bis 20 Minuten gut durchgerührt. Die untere wäßrige Phase wurde abgetrennt, einmal mit 200 ml Wasser gewaschen und danach das Lösungsmittel bei 300 mbar abdestilliert. Man erhielt nach Umkristallisation des Rückstandes 121,6 g an isomerem Dodecahydro-3a,6,6,9a-tetramethylnaphtho[2,1-b]furan (Formel Ib ; $R^1$, $R^2 = CH_3$), vom Fp. 60°C und $[\alpha]_D^{20}$ = +7,5°, was einer Ausbeute von 92% entspricht.

## Ansprüche

1. Verfahren zur Herstellung von alkylierten Dodecahydro-naphtho[2,1-b]furanen der allgemeinen Formel I

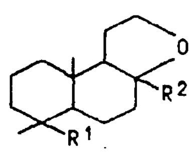

(I)

in der $R^1$ und $R^2$ die gleiche Bedeutung haben und für Wasserstoff oder eine Methylgruppe stehen, durch Umsetzung eines alkylierten 2-Hydroxy-decahydro-naphthalin-1-ethanols der Formel II

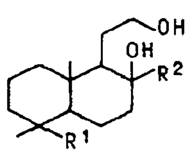

(II)

in der $R^1$ und $R^2$ die obengenannte Bedeutung besitzen, mit einem Sulfochlorid in einem organischen Lösungsmittel und in Gegenwart basischer Katalysatoren, dadurch gekennzeichnet, daß man die Cyclisierung in Gegenwart von konzentrierten wäßrigen Alkalihydroxiden und eines Phasentransferkatalysators durchführt.

2. Verfahren nach Anspruch 1, dadurch gekennzeichnet, daß man als Phasentransferkatalysator ein Tetraalkylammoniumsalz der allgemeinen Formel IV

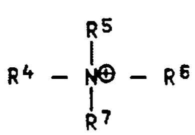

$X^{\ominus}$ , (IV), in der

$R^4$, $R^5$, $R^6$ und $R^7$ untereinander gleich oder verschieden sind und für Alkylreste mit 1 bis 22 Kohlenstoffatomen oder für funktionelle Gruppen, wie Hydroxyl–, Carbonamid– oder Ethergruppen enthaltende Alkylreste mit bis zu 25 Kohlenstoffatomen sowie für Phenylreste oder phenylsubstituierte Alkylreste mit bis zu 20 Kohlenstoffatomen stehen,

und $X^{\ominus}$ für ein Anion einer Säure wie $J^-$, $Cl^-$, $Br^-$, $(HSO_4)^-$, $(CN)^-$ oder $(BF_4)^-$ steht, verwendet.

3. Verfahren nach Anspruch 1, dadurch gekennzeichnet, daß man Trimethylbenzylammoniumchlorid oder

Tricaprylmethylammoniumchlorid als Phasentransferkatalysator verwendet.

4. Verfahren gemäß Anspruch 1, dadurch gekennzeichnet, daß man ein Tetraalkylphosphoniumsalz der allgemeinen Formel V

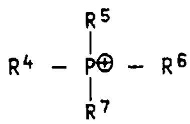 X⊖ , (V), in der

$R^4$, $R^5$, $R^6$, $R^7$ sowie X⊖ die für die Formel (IV) angegebene Bedeutung haben als Phasentransferkatalysator verwendet.

5. Verfahren gemäß Anspruch 1, dadurch gekennzeichnet, daß man Tri-n-octyl-methylphosphoniumiodid als Phasentransferkatalysator verwendet.

6. Verfahren nach Anspruch 1, dadurch gekennzeichnet, daß man als Sulfochlorid ein Sulfochlorid der allgemeinen Formel III

$$R^3 - SO_2Cl \quad (III)$$

verwendet, in der $R^3$ für einen $C_1$-$C_3$-Alkylrest oder einen Phenylrest steht, der in para-Stellung mit einer Methylgruppe, einem Chlor- oder Bromatom, oder aber einer Nitro-Gruppe, substituiert sein kann.

## Claims

1. A process for preparing an alkylated dodecahydro-naphtho(2,1-b]furan of the general formula I

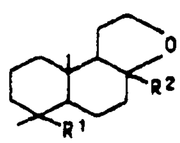 (I)

where $R^1$ and $R^2$ are identical and each is hydrogen or methyl, by reacting an alkylated 2-hydroxydecahydronaphthalene-1-ethanol of the formula II

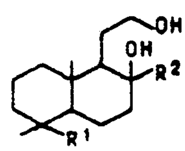 (II)

where $R^1$ and $R^2$ are each as defined above, with a sulfonyl chloride in an organic solvent and in the presence of a basic catalyst by performing the cyclization in the presence of a concentrated aqueous alkali metal hydroxide and of a phase transfer catalyst.

2. A process as claimed in claim 1, wherein the phase transfer catalyst used is a tetraalkylammonium salt of the general formula IV

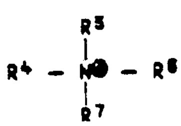 X⊖ (IV) where

$R^4$, $R^5$, $R^6$ and $R^7$ are identical to or different from one another and each is alkyl of from 1 to 22 carbon atoms or alkyl of up to 25 carbon atoms containing functional groups such as hydroxyl, carboxamide or ether groups, or is phenyl or phenyl-substituted alkyl of up to 20 carbon atoms, and X⊖ is an anion of an acid such as $I^-$, $Cl^-$, $Br^-$, $(HSO_4)^-$, $(CH)^-$ or $(BF_4)^-$.

3. A process as claimed in claim 1, wherein the phase transfer catalyst used is trimethylbenzylammonium

chloride or tricaprylomethylammonium chloride.

4. A process as claimed in claim 1, wherein the phase transfer catalyst used is a tetraalkylphosphonium salt of the general formula V

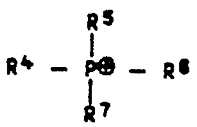 $X^{\ominus}$ (V) where

$R^4$, $R^5$, $R^6$, $R^7$ and $X^{\ominus}$ are as defined for the formula (IV).

5. A process as claimed in claim 1, wherein the phase transfer catalyst used is tri-n-octylmethylphosphonium iodide.

6. A process as claimed in claim 1, wherein the sulfonyl chloride used is a sulfonyl chloride of the general formula III

$$R^3\text{-}SO_2Cl \quad (III)$$

where $R^3$ is $C_1$-$C_3$-alkyl or phenyl which may be substituted in the para position by methyl, chlorine, bromine or nitro.

## Revendications

1. Procédé de préparation de dodécahydro-naphto(2,1-b)furannes alkylés de formule générale I

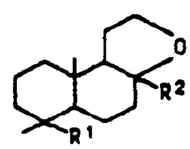 (I)

dans laquelle $R^1$ et $R^2$ ont la même signification et sont mis chacun pour un atome d'hydrogène ou un groupement méthyle, par réaction d'un 2-hydroxy-décahydro-naphtalène-1- éthanol alkylé de formule II

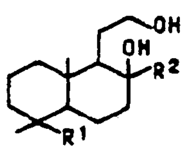 (II)

dans laquelle $R^1$ et $R^2$ ont les significations données précédemment, avec un sulfochlorure dans un solvant organique et en présence de catalyseurs basiques, caractérisé en ce qu'on effectue la cyclisation en présence d'hydroxydes alcalins aqueux concentrés et d'un catalyseur de transfert de phase.

2. Procédé selon la revendication 1, caractérisé en ce qu'on utilise, comme catalyseur de transfert de phase, un sel de tétraalkylammonium de formule générale IV

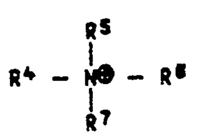 $X^{\ominus}$ , (IV),

dans laquelle $R^4$, $R^5$, $R^6$ et $R^7$ sont identiques ou différents les uns des autres et sont mis pour des restes alkyle à 1-22 atomes de carbone ou pour des restes alkyle renfermant jusqu'à 25 atomes de carbone et contenant des groupements fonctionnels tels que des groupements hydroxy, carbonamide ou éther, ainsi que pour des restes phényle ou des restes alkyle phénylsubstitués renfermant jusqu'à 20 atomes de carbone, et $X^{\ominus}$ est mis pour un anion d'un acide tel que $I^-$, $Cl^-$, $Br^-$, $(HSO_4)^-$, $(CN)^-$ ou $(BF_4)^-$.

3. Procédé selon la revendication 1, caractérisé en ce qu'on utilise, comme catalyseur de transfert de phase, du chlorure de triméthylbenzylammonium ou du chlorure de tricaprylméthylammonium.

4. Procédé selon la revendication 1, caractérisé en ce qu'on utilise, comme catalyseur de transfert de

phase, un sel de tétraalkylphosphonium de formule générale V

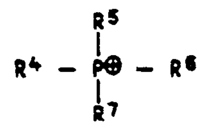 $X^{\ominus}$ , (V),

dans laquelle $R^4$, $R^5$, $R^6$, $R^7$ et $X^{\ominus}$ ont les significations données à propos de la formule (IV).

5. Procédé selon la revendication 1, caractérisé en ce qu'on utilise, comme catalyseur de transfert de phase, de l'iodure de tri-n-octylméthylphosphonium.

6. Procédé selon la revendication 1, caractérisé en ce qu'on utilise, comme sulfochlorure, un sulfochlorure de formule générale III

$$R^3\text{-}SO_2Cl \quad (III)$$

dans laquelle $R^3$ est mis pour un reste alkyle en $C_1$-$C_3$ ou un reste phényle qui peut être substitué, en position para, par un groupement méthyle, par un atome de chlore ou de brome ou encore par un groupement nitro.